# EUROPEAN PATENT APPLICATION

(11) **EP 3 970 800 A1**
(43) Date of publication of application: **23.03.2022**
(21) Application number: 20805404.9
(22) Date of filing: 08.05.2020
(51) Int. Cl.: A61P 35/00, A61P 39/06, A61P 43/00, A61P 17/18, A61Q 19/00, A61K 9/12, A61K 8/67, A61K 31/355, A61K 31/661

(54) **AGENT FOR PROTECTION AGAINST ATMOSPHERIC POLLUTANTS AND COMPOSITION FOR PROTECTION AGAINST ATMOSPHERIC POLLUTANTS**

(30) Priority: 13.05.2019 JP 2019090544
(71) Applicant: Showa Denko K.K., Tokyo 105-8518 (JP)
(72) Inventor: NAKAGAMI Yuko, Tokyo 105-8518 (JP); FUKADA Go, Tokyo 105-8518 (JP); KATO Eiko, Tokyo 105-8518 (JP)
(74) Representative: Tack, Jens
(86) International application number: PCT/JP2020/018693
(87) International publication number: WO 2020/230726

(57) **Abstract**

An agent for protection against atmospheric pollutants containing a tocopherol phosphate ester or a salt thereof as an active ingredient is provided. A composition for protection against atmospheric pollutants containing the above-mentioned agent for protection against atmospheric pollutants and a pharmaceutically acceptable carrier is further provided.

## Description

### [Technical Field]

The present invention relates to an agent for protection against atmospheric pollutants and a composition for protection against atmospheric pollutants.

Priority is claimed on Japanese Patent Application No. 2019-090544, filed May 13, 2019, the content of which is incorporated herein by reference.

### [Background Art]

In the atmosphere, chemical substances such as aromatic hydrocarbons such as dioxin and PCBs and polycyclic aromatic hydrocarbons (PAHs), oxidizing substances obtained when these chemical substances are oxidized by the action of ultraviolet rays, and the like are present. These substances have various influences on the human body. By inhaling these harmful substances into the human body by exhalation or absorbing them to the mucous membranes, various inflammations occur in the respiratory organs such as the lungs, the mucous membranes of the skin, and the internal organs, and eventually canceration of cells is caused. It has been reported that harmful substances such as PAHs contained in this atmospheric dust and oxides thereof bind to an aryl hydrocarbon receptor (AhR) present in cells, they are transferred into the cell nucleus to induce the expression of drug metabolizing genes, active oxygen (Reactive Oxygen Species: ROS) is produced in cells, and as a result, inflammations and invasion of cancer cells are caused (Non Patent Documents 1 and 2).

As agents for defense and agents for protection against atmospheric pollutants and suppression agents of symptoms relating to atmospheric pollution, substances having an antioxidative effect, plant-derived extracts, and the like which are for the purpose of suppressing ROS produced in cells have been reported (Patent Documents 1 to 3). In addition, substances that competitively bind to the AhR to suppress the influences of harmful substances have been reported (Non Patent Document 3). However, this effect is still insufficient.

In addition, as agents for protection for physically repelling atmospheric pollutants, formulations into which an oily coating agent is blended to cover the body surface, agents that capture irritation-causing substances contained in atmospheric pollutants, agents that suppress an oxidizing action, and the like have been proposed. However, although these agents for protection are effective in terms of repelling irritant substances, they are not agents that can protect cells from the in vivo action that may be caused.

### [Citation List]

### [Patent Documents]

[Patent Document 1]
   Japanese Unexamined Patent Application, First Publication No. 2016-88928
[Patent Document 2]
   Japanese Unexamined Patent Application, First Publication No. 2017-186276
[Patent Document 3]
   Japanese Patent No. 6456815

### [Non Patent Documents]

[Non Patent Document 1]
   Moorthy B et al., Polycyclic aromatic hydrocarbons: from metabolism to lung cancer. Toxicol Sci. 2015 May; 145(1):5-15.
[Non Patent Document 2]
   Nebert DW et al., Role of aryl hydrocarbon receptor-mediated induction of the CYP1 enzymes in environmental toxicity and cancer. J Biol Chem. 2004 Jun 4; 279(23):23847-50.
[Non Patent Document 3]
   Furue M et al., Antioxidative Phytochemicals Accelerate Epidermal Terminal Differentiation via the AHR-OVOL1 Pathway: Implications for Atopic Dermatitis. Acta Derm Venereol. 2018 Nov 5; 98(10):918-923.

### [Summary of Invention]

### [Technical Problem]

As described above, since PAHs in the atmosphere and oxidizing substances thereof cause various harmful actions such as inflammations on the biological body, a drug that protects cells from these atmospheric pollutants is required.

Accordingly, an object of the present invention is to provide an agent for protection against atmospheric pollutants which can protect cells from harmful actions due to atmospheric pollutants, and a composition for protection against atmospheric pollutants which contains the above-mentioned agent for protection against atmospheric pollutants.

### [Solution to Problem]

The present invention includes the following aspects.
(1) An agent for protection against atmospheric pollutants containing a tocopherol phosphate ester or a salt thereof as an active ingredient.
(2) The agent for protection against atmospheric pollutants according to (1), in which the tocopherol phosphate ester is an α-tocopherol phosphate ester.
(3) The agent for protection against atmospheric pollutants according to (1) or (2), in which the salt of the tocopherol phosphate ester is a sodium salt of the tocopherol phosphate ester.
(4) The agent for protection against atmospheric pollutants according to any one of (1) to (3), in which the agent for protection against atmospheric pollutants promotes expression of an NRF2 gene.
(5) The agent for protection against atmospheric pollutants according to any one of (1) to (4), in which the agent for protection against atmospheric pollutants suppresses production of active oxygen.
(6) A composition for protection against atmospheric pollutants containing: the agent for protection against atmospheric pollutants according to any one of (1) to (5); and a pharmaceutically acceptable carrier.
(7) The composition for protection against atmospheric pollutants according to (6), in which a total content of the tocopherol phosphate ester or the salt thereof is 0.1% to 2% by mass.
(8) A method for using the composition for protection against atmospheric pollutants according to (6) or (7), the method including using the composition for protection against atmospheric pollutants by spraying the composition on a body surface.
(9) A method for using the composition for protection against atmospheric pollutants according to (6) or (7), the method including using the composition for protection against atmospheric pollutants by applying the composition to a mucous membrane.

### [Advantageous Effects of Invention]

According to the present invention, it is possible to provide an agent for protection against atmospheric pollutants which can protect cells from harmful actions due to atmospheric pollutants, and a composition for protection against atmospheric pollutants which contains the above-mentioned agent for protection against atmospheric pollutants.

### [Description of Embodiments]

### [Agent for protection against atmospheric pollutants]

In one embodiment, the present invention provides an agent for protection against atmospheric pollutants containing a tocopherol phosphate ester or a salt thereof as an active ingredient.

The agent for protection against atmospheric pollutants of the present embodiment can be suitably used for protecting cells from atmospheric pollutants and suppressing harmful actions on cells from atmospheric pollutants. In the present specification, the "atmospheric pollutants" mean substances present in the atmosphere and causing a harmful action on the human body. Examples of the harmful action of atmospheric pollutants include an ROS production action, an action of suppressing expression of a nuclear factor erythroid 2-related factor-2 (NRF2) gene that controls the oxidative stress adaptation reaction, a cancer cell proliferation promoting action, and a cancer cell invasion promoting action. Examples thereof further include inflammations due to oxidative stress.

Examples of the atmospheric pollutants include substances included in Standard Reference Material 1648a supplied by the National Institute of Standards and Technology (NIST). Specific examples thereof include polycyclic aromatic hydrocarbons (PAHs), nitro-polycyclic aromatic hydrocarbons (nitro-PAHs), polychlorinated biphenyls (PCBs), and chlorine-based pesticides.

Examples of the polycyclic aromatic hydrocarbons include, but are not limited to, naphthalene, acenaphthene, phenanthrene, methylphenanthrene, anthracene, benzanthracene, dibenzanthracene, fluoranthene, benzofluoranthene, dibenzofluoranthene, pyrene, benzopyrene, dibenzopyrene, perylene, benzoperylene, indenoperylene, chrysene, benzochrysene, triphenylene, picene, coronene, and biphenyl.

Examples of the nitro-polycyclic aromatic hydrocarbons include compounds in which some of hydrogen atoms of the above-exemplified polycyclic aromatic hydrocarbons are substituted with nitro groups. Specific examples thereof include, but are not limited to, 1-nitronaphthalene, 2-nitronaphthalene, 3-nitroacetylene, 4-nitrophenanthrene, 9-nitrophenanthrene, 9-nitroanthracene, 1-nitropyrene, 2-nitropyrene, 4-nitropyrene, 2-nitrofluoranthene, 3-nitrofluoranthene, 8-nitrofluoranthene, 7-nitrobenzanthracene, and 6-nitrochrysene.

Examples of the polychlorinated biphenyls include, but are not limited to, dichlorobiphenyl, trichlorobiphenyl, tetrachlorobiphenyl, pentachlorobiphenyl, hexachlorobiphenyl, heptachlorobiphenyl, octachlorobiphenyl, nonachlorobiphenyl, and decachlorobiphenyl.

Examples of the chlorine-based pesticides include, but are not limited to, benzene hexachloride, hexachlorobenzene, chlordane, Mirex, and dichlorodiphenyltrichloroethane (DDT).

The atmospheric pollutant is not limited to one having a harmful action (ROS production, suppression of NRF2 gene expression, cancer proliferation, cancer invasion, and the like) alone, and may be one in which a plurality of substances act compositely to exert a harmful action.

As shown in Examples to be described later, when atmospheric pollutants are present, the expression of the NRF2 gene is suppressed, and ROS production is promoted. In addition, cancer cell proliferation and cancer cell invasion are promoted. The agent for protection against atmospheric pollutants of the present embodiment can effectively suppress such harmful actions induced by atmospheric pollutants. That is, the agent for protection against atmospheric pollutants of the present embodiment can increase the expression level of the NRF2 gene in the presence of atmospheric pollutants as compared to a case in which the above-mentioned agent for protection is not administered.

As shown in Examples to be described later, the agent for protection against atmospheric pollutants of the present embodiment can induce the expression of the NRF2 gene suppressed due to atmospheric pollutants. Accordingly, it can also be said that the agent for protection against atmospheric pollutants of the present embodiment is an NRF2 gene expression inducing agent. Alternatively, it can also be said that it is an upregulator of NRF2 gene expression suppressed due to atmospheric pollutants.

NRF2 (NCBI Gene ID: 4780) is a transcription factor having the basic leucine zipper structure and belongs to the CNC transcription factor group. NRF2 is a transcriptional regulatory factor of a group of genes containing an antioxidant response element (ARE) in a promoter, and controls the oxidative stress adaptation reaction. Examples of base sequences a human NRF2 gene include NM_001145412.3, NM_001145413.3, NM_001313900.1, NM_001313901.1, NM_001313902.1, NM_001313903.1, NM_001313904.1, and NM_006164.5 which are registered in the NCBI Reference Sequence database. The NRF2 gene is not limited to those having the above-mentioned sequence, and includes homologs thereof.

As shown in Examples to be described later, the agent for protection against atmospheric pollutants of the present embodiment can suppress the production of ROS induced by atmospheric pollutants. Accordingly, it can also be said that the agent for protection against atmospheric pollutants of the present embodiment is a ROS production suppression agent. That is, the agent for protection against atmospheric pollutants of the present embodiment can decrease the production amount of ROS in the presence of atmospheric pollutants as compared to a case in which the above-mentioned agent for protection is not administered.

As shown in Examples to be described later, the agent for protection against atmospheric pollutants of the present embodiment can suppress the proliferation of cancer cells promoted by atmospheric pollutants. Accordingly, it can also be said that the agent for protection against atmospheric pollutants of the present embodiment is a cancer cell proliferation suppression agent. That is, the agent for protection against atmospheric pollutants of the present embodiment can suppress the proliferation of cancer cells in the presence of atmospheric pollutants as compared to a case in which the above-mentioned agent for protection is not administered.

As shown in Examples to be described later, the agent for protection against atmospheric pollutants of the present embodiment can suppress the invasion of cancer cells promoted by atmospheric pollutants. Accordingly, it can also be said that the agent for protection against atmospheric pollutants of the present embodiment is a cancer cell invasion suppression agent. That is, the agent for protection against atmospheric pollutants of the present embodiment can suppress the invasion of cancer cells in the presence of atmospheric pollutants as compared to a case in which the above-mentioned agent for protection is not administered.

### (Tocopherol phosphate ester and salt thereof)

The agent for protection against atmospheric pollutants of the present embodiment contains a tocopherol phosphate ester or a salt thereof as an active ingredient.

Examples of the tocopherol phosphate ester include a compound represented by General Formula (1).

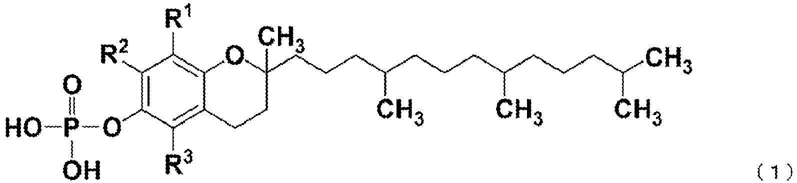

### [In the formula, R¹, R², and R³ each independently represent a hydrogen atom or a methyl group.]

As the tocopherol phosphate ester, α-tocopherol phosphate ester (R¹, R², R³ = CH₃), β-tocopherol phosphate ester (R¹, R³ = CH₃, R² = H), γ-tocopherol phosphate ester (R¹, R² = CH₃, R³ = H), δ-tocopherol phosphate ester (R¹ = CH₃, R², R³ = H), ζ₂-tocopherol phosphate ester (R², R³ = CH₃, R¹ = H), η-tocopherol phosphate ester (R² = CH₃, R¹, R³ = H), and the like are present according to the type of R¹, R², and R³ in General Formula (1).

The tocopherol phosphate ester is not particularly limited, and may be any of these tocopherol phosphate esters. Among these, α-tocopherol phosphate ester and γ-tocopherol phosphate ester are preferable, and α-tocopherol phosphate ester is more preferable.

Since the compound represented by General Formula (1) has an asymmetrical carbon atom at the 2-position of a chroman ring, stereoisomeric forms of the d-form and the l-form, and the dl-form are present. The tocopherol phosphate ester may be any of these stereoisomeric forms, but the dl-form is preferable.

Among the above, as the tocopherol phosphate ester, dl-α-tocopherol phosphate ester and dl-γ-tocopherol phosphate ester are preferable, and dl-α-tocopherol phosphate ester is more preferable.

The salt of the tocopherol phosphate ester is not particularly limited, and examples thereof include salts of inorganic bases and salts of organic bases.

Examples of the salts of inorganic bases include alkali metal salts such as sodium salts and potassium salts; alkaline earth metal salts such as calcium salts and magnesium salts; aluminum salts; ammonium salts; and zinc salts.

Examples of the salts of organic bases include alkylammonium salts and salts of basic amino acids.

Among the above, as the salt of the tocopherol phosphate ester, alkali metal salts are preferable, and sodium salts are more preferable. Among alkali metal salts of the tocopherol phosphate ester, particularly sodium salts have advantages that they are highly soluble in water and they are easily handled because of their property of being a powder.

Examples of preferred forms of the tocopherol phosphate ester include alkali metal salts (for example, sodium salts) of the compound represented by General Formula (1), alkali metal salts (for example, sodium salts) of α-tocopherol phosphate ester, alkali metal salts (for example, sodium salts) of γ-tocopherol phosphate ester, alkali metal salts (for example, sodium salts) of dl-α-tocopherol phosphate ester, and alkali metal salts (for example, sodium salt) of dl-γ-tocopherol phosphate ester.

Sodium salts of dl-α-tocopherol phosphate ester are commercially available from Showa Denko K.K. under the product name of TPNa (registered trademark) (display name: Na tocopheryl phosphate). The above-mentioned TPNa is exemplified as a preferable example of a tocopherol phosphate ester.

In the agent for protection against atmospheric pollutants of the present embodiment, one kind selected from a tocopherol phosphate ester and a salt thereof may be used alone, or two or more thereof may be used in combination. A composition for protection against atmospheric pollutants of the present embodiment preferably contains salts of tocopherol phosphate ester, and it is more preferable to use alkali metal salts (for example, sodium salts) of tocopherol phosphate ester alone.

The tocopherol phosphate ester or a salt thereof can be manufactured by a known manufacturing method, for example, methods disclosed in Japanese Unexamined Patent Application, First Publication No. S59-44375, WO97/14705, and the like. For example, the tocopherol phosphate ester can be obtained by causing a phosphorylating agent such as phosphorus oxychloride to act on tocopherol dissolved in a solvent and appropriately purifying after completion of the reaction. Furthermore, salts of the obtained tocopherol phosphate ester can be obtained by neutralizing the tocopherol phosphate ester with a metal oxide such as magnesium oxide, a metal hydroxide such as sodium hydroxide, ammonium hydroxide or alkylammonium hydroxide, or the like.

Hereinafter, the tocopherol phosphate ester and a salt thereof may be collectively referred to as "tocopherol phosphate ester and the like".

The agent for protection against atmospheric pollutants of the present embodiment can be used by administering itself to a patient for the purpose of protecting cells from atmospheric pollutants. Furthermore, the agent for protection against atmospheric pollutants of the present embodiment can be used by being blended into a pharmaceutical and a cosmetic product for the purpose of imparting the function of protecting cells from atmospheric pollutants. Furthermore, it may be used by being blended into a composition for protection against atmospheric pollutants to be described later.

The agent for protection against atmospheric pollutants of the present embodiment may be used by being administered to a patient in a region in which the concentration of atmospheric pollutants is high for preventing inflammations and carcinogenesis due to atmospheric pollutants. In addition, the agent for protection against atmospheric pollutants of the present embodiment may be used by being administered to a patient who has developed inflammations and/or cancer for suppressing the exacerbation of inflammations due to atmospheric pollutants and/or the proliferation and/or invasion of cancer cells.

The agent for protection against atmospheric pollutants of the present embodiment can be administered to a patient by the same method as that for the composition for protection against atmospheric pollutants to be described later, and is preferably administered transdermally.

### [Composition for protection against atmospheric pollutants]

In one embodiment, the present invention provides a composition for protection against atmospheric pollutants containing the above-mentioned agent for protection against atmospheric pollutants and a pharmaceutically acceptable carrier.

The composition for protection against atmospheric pollutants of the present embodiment can be manufactured by mixing the above-mentioned agent for protection against atmospheric pollutants, a pharmaceutically acceptable carrier, and optionally other components and formulating them according to a general method (for example, a method described in the Japanese Pharmacopoeia).

In the present specification, a "pharmaceutically acceptable carrier" means a carrier that does not inhibit a physiological activity of an active ingredient and does not exhibit substantial toxicity with respect to its administration target. The phrase "does not exhibit substantial toxicity" means that this component does not exhibit toxicity with respect to an administration target in a generally used dosage. The pharmaceutically acceptable carrier is not particularly limited, and examples thereof includes excipients, binders, disintegrants, lubricants, emulsifiers, stabilizers, diluents, solvents for injections, oily bases, moisturizers, touch sensation improvers, surfactants, polymers, thickeners, gelling agents, solvents, propellants, antioxidants, reducing agents, oxidizing agents, chelating agents, acids, alkali, powders, inorganic salts, water, metal-containing compounds, unsaturated monomers, polyhydric alcohols, polymer additives, adjuvants, wetting agents, tackifiers, oily raw materials, liquid matrices, fat-soluble substances, and polymer carboxylate salts. Specific examples of these components include those described in PCT International Publication No. WO2016/076310. In addition, specific examples of the polymers, thickeners, and gelling agents include methacryloyloxyethyl phosphorylcholine, butyl methacrylate, and polymers thereof. These pharmaceutically acceptable carriers may be used alone or in combination of two or more kinds thereof.

The other components are not particularly limited, but examples thereof include preservatives, antibacterial agents, ultraviolet absorbents, whitening agents, vitamins and derivatives thereof, antiphlogistics, anti-inflammatory agents, hair growth drugs, blood circulation promoters, stimulants, hormones, anti-wrinkle agents, anti-aging agents, tightening agents, cooling sensation agents, warming sensation agents, wound healing promoters, irritation relieving agents, analgesics, cell-activating agents, plant extracts, animal extracts, microbial extracts, seed oils, antipruritic agents, keratin exfoliating and dissolving agents, antiperspirants, refreshing agents, astringents, enzymes, nucleic acids, fragrances, colorants, coloring agents, dyes, pigments, anti-inflammatory analgesics, antifungals, antihistamines, hypnotic sedatives, tranquilizers, antihypertensives, antihypertensive diuretics, antibiotics, anesthetics, antibacterial substances, antiepileptic agents, coronary vasodilators, herbal medicines, anti-itch drugs, keratin softening and exfoliating agents, ultraviolet blockers, antiseptic disinfectants, antioxidant substances, pH adjusters, additives, and metal soaps. Specific examples of these components include those described in PCT International Publication No. WO2016/076310. In addition, specific examples of the plant extracts include Lapsana communis flowers/leaves/stems, and Camellia sinensis leaves. Specific examples of the seed oils include a Moringa oleifera seed oil. Specific examples of the fragrances include perillaldehyde. The other components may be used alone or in combination of two or more kinds thereof.

The composition for protection against atmospheric pollutants of the present embodiment can contain a therapeutically effective amount of the above-mentioned agent for protection against atmospheric pollutants. The "therapeutically effective amount" means the amount of a drug effective for treating or preventing diseases of patients. The therapeutically effective amount may vary depending on a disease state, age, sex, body weight, and the like of an administration target. In the composition for protection against atmospheric pollutants of the present embodiment, the therapeutically effective amount of the above-mentioned agent for protection against atmospheric pollutants may be an amount in which the tocopherol phosphate ester and the like in the agent for protection can suppress harmful actions due to atmospheric pollutants. For example, the therapeutically effective amount of the above-mentioned agent for protection against atmospheric pollutants in the composition for protection against atmospheric pollutants of the present embodiment may be 0.01% to 20% by mass for example, may be 0.1% to 10% by mass for example, may be 0.1% to 5% by mass for example, may be 0.1% to 3% by mass for example, may be 0.1% to 2% by mass for example, may be 0.3% to 2% by mass for example, or may be 0.6% to 1.5% by mass for example as the total content of the tocopherol phosphate ester and the like in the composition.

The content of the tocopherol phosphate ester and the like in the above-mentioned composition for protection against atmospheric pollutants means the content of one kind of tocopherol phosphate ester and the like when this compound is contained alone, and means the total content of two or more kinds of tocopherol phosphate esters and the like when these compounds are contained in combination.

The composition for protection against atmospheric pollutants of the present embodiment may be a pharmaceutical composition or a cosmetic preparation.

### (Pharmaceutical composition)

In one embodiment, the present invention provides a pharmaceutical composition for protecting cells from atmospheric pollutants containing the above-mentioned agent for protection against atmospheric pollutants and a pharmaceutically acceptable carrier.

In the pharmaceutical composition of the present embodiment, the pharmaceutically acceptable carrier is not particularly limited, and carriers generally used for pharmaceuticals can be used in addition to the carriers exemplified above. For example, it is possible to use general raw materials described in the Japanese Pharmacopoeia, the Japanese Pharmaceutical Codex, Japanese Pharmaceutical Excipients 2013 (Yakuji Nippo, Ltd., 2013), the Japanese Pharmaceutical Excipients Directory 2016 (edited by International Pharmaceutical Excipients Council Japan, Yakuji Nippo, Ltd., 2016), Handbook of Pharmaceutical Excipients, 7th edition (Pharmaceutical Press, 2012), and the like. These pharmaceutically acceptable carriers may be used alone or in combination of two or more kinds thereof.

The pharmaceutical composition of the present embodiment may contain other components in addition to the above-mentioned agent for protection against atmospheric pollutants and the pharmaceutically acceptable carriers. The other components are not particularly limited, and general pharmaceutical additives can be used. Furthermore, as the other components, it is also possible to use an active ingredient other than the above-mentioned agent for protection against atmospheric pollutants. As pharmaceutical additives and active ingredients as the other components, in addition to those exemplified above, it is possible to use general raw materials described in, for example, the Japanese Pharmacopoeia, the Japanese Pharmaceutical Codex, Japanese Pharmaceutical Excipients 2013 (Yakuji Nippo, Ltd., 2013), the Japanese Pharmaceutical Excipients Directory 2016 (edited by International Pharmaceutical Excipients Council Japan, Yakuji Nippo, Ltd., 2016), Handbook of Pharmaceutical Excipients, 7th edition (Pharmaceutical Press, 2012), and the like. The other components may be used alone or in combination of two or more kinds thereof.

A dosage form of the pharmaceutical composition of the present embodiment is not particularly limited, and it can be a dosage form generally used for pharmaceutical preparations. Examples thereof include orally administered dosage forms such as tablets, coated tablets, pills, powders, granules, capsules, solutions, suspensions, and emulsions; and parenterally administered dosage forms such as injections, suppositories, external preparations for the skin, and nasal drops. The pharmaceutical composition in these dosage forms can be formulated according to a general method (for example, a method described in the Japanese Pharmacopoeia).

As the pharmaceutical composition of the present embodiment, an external preparation for the skin or a nasal drop is preferable. More specific examples of the external preparation for skin include dosage forms such as creams, lotions, packs, foams, skin cleansers, extracts, plasters, ointments, spirits, suspensions, tinctures, tapes, poultices, liniments, aerosols, sprays, and gels.

A method for administering the pharmaceutical composition of the present embodiment is not particularly limited, and the pharmaceutical composition can be administered by a method generally used as a method for administering pharmaceuticals. For example, it may be administered orally as tablets, coated tablets, pills, powders, granules, capsules, solutions, suspensions, emulsions, and the like; it may be administered intravenously, intraarterially, intramuscularly, intradermally, subcutaneously, intraperitoneally, and the like as an injections, as infusion preparations, and the like alone, or as a mixture with common infusions such as a glucose solution and Ringer's solution; it may be administered rectally as suppositories; it may be administered to skin as external preparations for the skin; or it may be administered intranasally as nasal drops. In a preferred aspect, the pharmaceutical composition of the present embodiment is applied, affixed, or sprayed to an affected area as external preparations for the skin. Alternatively, it is administered intranasally as nasal drops.

The dosage of the pharmaceutical composition of the present embodiment can be a therapeutically effective amount. The therapeutically effective amount may be appropriately determined according to symptoms, body weight, age, sex, and the like of a patient, a dosage form of the pharmaceutical composition, an administration method, and the like. For example, in the case of oral administration, the dosage of the pharmaceutical composition of the present embodiment may be 0.01 to 500 mg per unit of a dosage form as the tocopherol phosphate ester and the like; in the case of injections, the dosage may be 0.02 to 250 mg per unit of a dosage form as the tocopherol phosphate ester and the like; in the case of suppositories, the dosage may be 0.01 to 500 mg per unit of a dosage form as the tocopherol phosphate ester and the like; and the like. In the case of external preparations for the skin or nasal drops, the dosage of the pharmaceutical composition of the present embodiment may be 0.15 to 500 mg per unit of a dosage form as the tocopherol phosphate ester and the like for example, may be 0.15 to 300 mg for example, may be 0.15 to 200 mg for example, and may be 0.2 to 100 mg for example.

The administration interval of the pharmaceutical composition of the present embodiment may be appropriately determined according to symptoms, body weight, age, sex, and the like of a patient, a dosage form of the pharmaceutical composition, an administration method, and the like. For example, it may be once a day, about 2 to 3 times a day, or the like.

The pharmaceutical composition of the present embodiment can be used by being administered to, for example, a patient having inflammations due to atmospheric pollutants for suppressing the exacerbation of inflammations. In addition, the pharmaceutical composition of the present embodiment can be used by being administered to a cancer patient for suppressing the proliferation and/or invasion of cancer cells induced by atmospheric pollutants.

In urban areas, people are in daily contact with atmospheric pollutants frequently, and thus are exposed to a risk of being affected by harmful actions due to atmospheric pollutants. In addition, patients having inflammations are at a risk of the exacerbation of inflammations upon contact with atmospheric pollutants. Furthermore, cancer patients are at a risk of promoting the proliferation and/or invasion of cancer cells upon contact with atmospheric pollutants. Therefore, it is possible to reduce the risk of the exacerbation of inflammations and cancer symptoms due to atmospheric pollutants by administering the pharmaceutical composition of the present embodiment to patients with inflammations and cancer patients.

Alternatively, in regions in which atmospheric pollutants are present, the pharmaceutical composition of the present embodiment can be used by being prophylactically administered to patients to prevent the onset of inflammations. Furthermore, it can be used by suppressing proliferation of cancerous cells due to contact with atmospheric pollutants to prevent the onset of cancer.

### (Cosmetic preparation)

In one embodiment, the present invention provides a cosmetic preparation for protection against atmospheric pollutants containing the above-mentioned agent for protection against atmospheric pollutants and a pharmaceutically acceptable carrier.

In the cosmetic preparation of the present embodiment, the pharmaceutically acceptable carrier is not particularly limited, and carriers generally used for cosmetic preparations can be used in addition to those exemplified above. For example, it is possible to use general raw materials described in the Japanese Standards of Cosmetic Ingredients, Second Edition, Supplements (edited by the Pharmaceutical and Medical Device Regulatory Science Society of Japan, Yakuji Nippo, Ltd., 1984); the Japanese Cosmetic Ingredients Codex (supervisorily edited by the Examination Division of the Pharmaceutical Affairs Bureau, Yakuji Nippo, Ltd., 1993); Supplement to the Japanese Cosmetic Ingredients Codex (supervisorily edited by the Examination Division of the Pharmaceutical Affairs Bureau, Yakuji Nippo, Ltd., 1993); the Comprehensive Licensing Standards of Cosmetics by Category (supervisorily edited by the Examination Division of the Pharmaceutical Affairs Bureau, Yakuji Nippo, Ltd., 1993); the Dictionary of Cosmetic Ingredients (Nikko Chemicals Co., Ltd., 1991); International Cosmetic Ingredient Dictionary and Handbook 2002, Ninth Edition, Vol. 1 to 4, CTFA; and the like. These pharmaceutically acceptable carriers may be used alone or in combination of two or more kinds thereof.

The cosmetic preparation of the present embodiment may contain other components in addition to the agent for protection against atmospheric pollutants and the pharmaceutically acceptable carriers. The other components are not particularly limited, and general additives for cosmetic products can be used. Furthermore, as the other components, it is also possible to use an active ingredient other than the above-mentioned agent for protection against atmospheric pollutants. As additives for cosmetic products and active ingredients as the other components, in addition to those exemplified above, it is possible to use general raw materials described in, for example, the Japanese Standards of Cosmetic Ingredients, Second Edition, Supplements (edited by the Pharmaceutical and Medical Device Regulatory Science Society of Japan, Yakuji Nippo, Ltd., 1984); the Japanese Cosmetic Ingredients Codex (supervisorily edited by the Examination Division of the Pharmaceutical Affairs Bureau, Yakuji Nippo, Ltd., 1993); Supplement to the Japanese Cosmetic Ingredients Codex (supervisorily edited by the Examination Division of the Pharmaceutical Affairs Bureau, Yakuji Nippo, Ltd., 1993); the Comprehensive Licensing Standards of Cosmetics by Category (supervisorily edited by the Examination Division of the Pharmaceutical Affairs Bureau, Yakuji Nippo, Ltd., 1993); the Dictionary of Cosmetic Ingredients (Nikko Chemicals Co., Ltd., 1991); International Cosmetic Ingredient Dictionary and Handbook 2002, Ninth Edition, Vol. 1 to 4, CTFA; and the like. The other components may be used alone or in combination of two or more kinds thereof.

The form of the cosmetic preparation of the present embodiment is not particularly limited, and it can be a form generally used for cosmetic preparations. Examples thereof include hair cosmetic preparations such as shampoos, hair conditioners, and hairdressing agents; basic cosmetic preparations such as facial cleansers, cleansing agents, skin toners, emulsions, lotions, creams, gels, sunscreens, packs, masks, and serums; makeup cosmetic preparations such as foundations, makeup primers, lipsticks, lip glosses, and blushers; body cosmetic preparations such as body cleansers, body powders, and deodorant cosmetics; and the like. These cosmetic preparations can be manufactured according to a general method. Among these, the cosmetic preparation of the present embodiment is preferably a cosmetic preparation in a form in which it is applied or attached to the skin as an external preparation for the skin. Preferable examples thereof include skin toners, emulsions, lotions, creams, gels, sunscreens, packs, masks, serums, foundations, makeup primers, and the like.

A dosage form of the cosmetic preparation of the present embodiment is not particularly limited, but examples thereof include emulsified types such an oil-in-water (O/W) type, a water-in-oil (W/O) type, a W/O/W type, and an O/W/O type, emulsified polymer types, oily types, solid types, liquid types, kneaded types, stick types, volatile oil types, powder types, jelly types, gel types, paste types, cream types, sheet types, film types, mist types, spray types, aerosol types, multilayer types, foam types, flake types, and the like.

The use amount of the cosmetic preparation of the present embodiment is not particularly limited, but it can be an amount effective for protecting cells from harmful actions due to atmospheric pollutants. For example, the use amount of the cosmetic preparations of the present embodiment may be 0.15 to 500 mg per use as the amount of the tocopherol phosphate ester and the like, and it may be 0.15 to 300 mg for example, may be 0.15 to 200 mg for example, and may be 0.2 to 100 mg for example.

The use interval of the cosmetic preparation of the present embodiment is not particularly limited, but it can be once a day, about 2 to 3 times a day, or the like, for example.

The cosmetic preparation the present embodiment may be used by patients with inflammations, cancer patients, and the like in urban areas, in which the concentration of atmospheric pollutants is high, and the like to reduce harmful actions due to atmospheric pollutants. Alternatively, it may be used in routine skin care and makeup to prevent inflammations and the onset of cancer due to atmospheric pollutants in regions or seasons in which the distribution concentration of atmospheric pollutants is high.

### [Other embodiments]

In one embodiment, the present invention provides a method for protecting cells from atmospheric pollutants, the method including a step of administering a tocopherol phosphate ester or a salt thereof to a mammal.

In one embodiment, the present invention provides a method for promoting the expression of an NRF2 gene in the presence of atmospheric pollutants, the method including a step of administering a tocopherol phosphate ester or a salt thereof to a mammal.

In one embodiment, the present invention provides a method for suppressing ROS production in the presence of atmospheric pollutants, the method including a step of administering a tocopherol phosphate ester or a salt thereof to a mammal.

In one embodiment, the present invention provides a method for suppressing cancer cell proliferation in the presence of atmospheric pollutants, the method including a step of administering a tocopherol phosphate ester or a salt thereof to a mammal.

In one embodiment, the present invention provides a method for suppressing cancer cell invasion in the presence of atmospheric pollutants, the method including a step of administering a tocopherol phosphate ester or a salt thereof to a mammal.

In one embodiment, the present invention provides a tocopherol phosphate ester or a salt thereof for protecting cells from atmospheric pollutants.

In one embodiment, the present invention provides a tocopherol phosphate ester or a salt thereof for promoting the expression of an NRF2 gene in the presence of atmospheric pollutants.

In one embodiment, the present invention provides a tocopherol phosphate ester or a salt thereof for suppressing ROS production in the presence of atmospheric pollutants.

In one embodiment, the present invention provides a tocopherol phosphate ester or a salt thereof for suppressing cancer cell proliferation in the presence of atmospheric pollutants.

In one embodiment, the present invention provides a tocopherol phosphate ester or a salt thereof for suppressing cancer cell invasion in the presence of atmospheric pollutants.

In one embodiment, the present invention provides use of a tocopherol phosphate ester or a salt thereof for manufacturing an agent for protection against atmospheric pollutants.

In one embodiment, the present invention provides use of a tocopherol phosphate ester or a salt thereof for manufacturing an agent for promoting NRF2 gene expression in the presence of atmospheric pollutants.

In one embodiment, the present invention provides use of a tocopherol phosphate ester or a salt thereof for manufacturing an agent for suppressing ROS production in the presence of atmospheric pollutants.

In one embodiment, the present invention provides use of a tocopherol phosphate ester or a salt thereof for manufacturing an agent for suppressing cancer cell proliferation in the presence of atmospheric pollutants.

In one embodiment, the present invention provides use of a tocopherol phosphate ester or a salt thereof for manufacturing an agent for suppressing cancer cell invasion in the presence of atmospheric pollutants.

In one embodiment, the present invention provides use of a tocopherol phosphate ester or a salt thereof for manufacturing a composition for protection against atmospheric pollutants.

In one embodiment, the present invention provides use of a tocopherol phosphate ester or a salt thereof for manufacturing a composition for promoting NRF2 gene expression in the presence of atmospheric pollutants.

In one embodiment, the present invention provides use of a tocopherol phosphate ester or a salt thereof for manufacturing a composition for suppressing ROS production in the presence of atmospheric pollutants.

### [Examples]

Hereinafter, the present invention will be described with reference to examples, but the present invention is not limited to the following examples.

In the following experimental examples, as the following Na tocopherol phosphate, TPNa (registered trademark) (manufactured by Showa Denko K.K.), which is sodium dl-α-tocopheryl phosphate, was used.

### [Experimental Example 1]

### (Effect of promoting expression of NRF2 gene)

The effect of Na tocopherol phosphate on promoting the expression of an NRF2 gene in normal human epidermal keratinocytes (NHEK, manufactured by KURABO INDUSTRIES LTD.) was measured under the following conditions.

The NHEK cells were seeded in a HuMedia KG2 medium manufactured by KURABO INDUSTRIES LTD. at the seeding density of 10000 cells/cm², and cultured for 24 hours under the conditions of 37°C and 5% CO₂. Next, a sample, in which Na tocopherol phosphate or tocopherol acetate was dissolved in an aqueous solution of 0.05% (V/V) ethanol, was added to the culture medium so that the final concentration of Na tocopherol phosphate or tocopherol acetate was 10 µM, and culturing was performed for 24 hours. Furthermore, one obtained by adding 5 µL of the aqueous solution of 0.05% (V/V) ethanol to the culture medium and culturing for 24 hours was also prepared. Thereafter, 0.1 mL of a DMSO solution of atmospheric dust (NIST 1648a) was added per 100 mL of the culture medium so that the final concentration of the atmospheric dust in the culture medium was 500 µg/mL, and culturing was further performed for 48 hours. Thereafter, the NHEK cells were recovered to extract total RNA using Nucleospin^{™} RX (Takara Bio Inc.). From the obtained RNAs, cDNA was synthesized using a PrimeScript (registered trademark) RT Master Mix (Takara Bio Inc.). Using this cDNA as a template, the expression level of the NRF2 gene was quantitatively determined by quantitative real-time PCR using a primer specific to the NRF2 gene (Perfect Real Time Primer, manufactured by Takara Bio Inc.). As an internal standard gene, the expression level of GAPDH was quantitatively determined (primer used: Perfect Real Time Primer, manufactured by Takara Bio Inc.), and the expression level of the NRF2 gene was standardized based on the expression level of GAPDH. One to which atmospheric dust was not added was used as a control.

Table 1 shows the results. Table 1 shows the expression level of the NRF2 gene in the atmospheric dust-added group as a relative expression level when the expression level of the NRF2 gene in the control to which atmospheric dust was not added was 1. In the Na tocopherol phosphate-added group, the expression of the NRF2 gene was promoted as compared to the 0.05% ethanol-added group and the tocopherol acetate-added group. From these results, it was confirmed that the Na tocopherol phosphate has the effect of restoring the expression of the NRF2 gene suppressed due to atmospheric dust.

**[Table 1]**

| Sample | | Relative gene expression level |
|---|---|---|
| | | NRF2 |
| Atmospheric dust not added | 0.05% Ethanol | 1.00 |
| Atmospheric dust added | 0.05% Ethanol | 0.67 |
| | Na tocopherol phosphate | 0.94 |
| | Tocopherol acetate | 0.71 |

### [Experimental Example 2]

### (Effect of suppressing ROS production)

The effect of Na tocopherol phosphate on suppressing ROS production in normal human epidermal keratinocytes (NHEK, manufactured by KURABO INDUSTRIES LTD.) was measured under the following conditions.

The NHEK cells were seeded in a HuMedia KG2 medium manufactured by KURABO INDUSTRIES LTD. at the seeding density of 10000 cells/cm², and cultured for 24 hours under the conditions of 37°C and 5% CO₂. Next, a sample, in which Na tocopherol phosphate or tocopherol acetate was dissolved in an aqueous solution of 0.05% (V/V) ethanol, was added to the culture medium so that the final concentration of Na tocopherol phosphate or tocopherol acetate was 10 µM, and culturing was performed for 24 hours under the conditions of 37°C and 5% CO₂. Furthermore, one obtained by adding 1 µL of the aqueous solution of 0.05% (V/V) ethanol to the culture medium and culturing for 24 hours was also prepared. Thereafter, 0.1 mL of a DMSO solution of atmospheric dust (NIST 1648a) was added per 100 mL of the culture medium so that the final concentration of the atmospheric dust in the culture medium was 500 µg/mL, and culturing was further performed for 48 hours. Thereafter, the ROS production amount was measured using a ROS assay kit (manufactured by OZ BIOSCIENCES). After washing the cells from which the culture medium was removed with a phosphate buffer solution (PBS, manufactured by FUJIFILM Wako Pure Chemical Corporation), 100 µL of dichlorofluorescein diacetate attached to the ROS assay kit was added to each group of the cells, and the cells were left to stand at 37°C for 30 minutes while being shielded from light. After washing with PBS again, 100 µL of PBS was added, and the fluorescence intensity at an excitation wavelength of 485 nm/an absorption wavelength of 535 nm was measured with a microplate reader i-Control (manufactured by Tecan Group Ltd.). One to which atmospheric dust was not added was used as a control.

Table 2 shows the results. Table 2 shows the ROS production amount in the atmospheric dust-added group as a relative amount when the fluorescence intensity in the control to which atmospheric dust was not added was 1. In the Na tocopherol phosphate-added group, the ROS production amount was reduced as compared to the 0.05% ethanol-added group and the tocopherol acetate-added group. From these results, it was confirmed that the Na tocopherol phosphate has a high suppression effect on the ROS production induced by atmospheric dust.

**[Table 2]**

| Sample | | Relative ROS production amount |
|---|---|---|
| Atmospheric dust not added | 0.05% Ethanol | 1.00 |
| Atmospheric dust added | 0.05% Ethanol | 1.54 |
| | Na tocopherol phosphate | 1.24 |
| | Tocopherol acetate | 1.67 |

### [Experimental Example 3]

### (Effect of suppressing proliferation of cancer cells)

The effect of Na tocopherol phosphate on suppressing the proliferation of cancer cells in a cell line derived from Lewis lung carcinoma (LLC, JCRB Cell Bank) was measured under the following conditions.

The LLC cells were seeded at the seeding density of 50000 cells/cm² in a culture medium in which a Ham F10 medium and an L15 medium (both manufactured by Sigma-Aldrich) were mixed at the ratio of 3:7 (volume ratio), and cultured for 24 hours under the conditions of 37°C and 5% CO₂. Next, a sample, in which Na tocopherol phosphate or tocopherol acetate was dissolved in an aqueous solution of 0.05% (V/V) ethanol, was added to the culture medium so that the final concentration of Na tocopherol phosphate or tocopherol acetate was 10 µM, and culturing was performed for 24 hours. Furthermore, one obtained by adding 1 µL of the aqueous solution of 0.05% (V/V) ethanol to the culture medium and culturing for 24 hours was also prepared. Thereafter, 0.1 mL of a DMSO solution of atmospheric dust (NIST 1648a) was added per 100 mL of the culture medium so that the final concentration of the atmospheric dust in the culture medium was 500 µg/mL, and culturing was further performed for 48 hours. Thereafter, the culture medium was replaced with a medium containing 10% (V/V) of WST-8 of Nacalai Tesque Inc., and after further culturing for 3 hours, the absorbance at the wavelength of 450 nm was measured with a microplate reader i-Control (manufactured by Tecan Group Ltd.). One to which atmospheric dust was not added was used as a control.

Table 3 shows the results. Table 3 shows the cell proliferation amount in the atmospheric dust-added group as a relative amount when the absorbance in the control to which atmospheric dust was not added was 1. In the Na tocopherol phosphate-added group, the cell proliferation amount of cancer cells was reduced as compared to the 0.05% ethanol-added group and the tocopherol acetate-added group. From these results, it was confirmed that the Na tocopherol phosphate has a high suppression effect on the cell proliferation of cancer cells accelerating due to atmospheric dust.

**[Table 3]**

| Sample | | Relative cell proliferation amount |
|---|---|---|
| Atmospheric dust not added | 0.05% Ethanol | 1.00 |
| Atmospheric dust added | 0.05% Ethanol | 4.25 |
| | Na tocopherol phosphate | 2.33 |
| | Tocopherol acetate | 3.98 |

### [Experimental Example 4]

### (Effect of suppressing invasion of cancer cells)

The effect of Na tocopherol phosphate on suppressing the invasion of cells in a cell line derived from Lewis lung carcinoma (LLC, JCRB Cell Bank) was measured under the following conditions. A CytoSelect invasion assay kit manufactured by Cell Biolabs, Inc. was used for a test.

The LLC cells were seeded in a chamber plate for invasion tests attached to the above-mentioned invasion assay kit so that the concentration was 100000 cells/mL using a culture medium in which a Ham F10 medium and an L15 medium (both manufactured by Sigma-Aldrich) were mixed at the ratio of 3:7 (volume ratio), and cultured for 24 hours under the conditions of 37°C and 5% CO₂. Next, a sample, in which Na tocopherol phosphate or tocopherol acetate was dissolved in an aqueous solution of 0.05% (V/V) ethanol, was added to the culture medium so that the final concentration of Na tocopherol phosphate or tocopherol acetate was 10 µM, and culturing was further performed for 24 hours under the conditions of 37°C and 5% CO₂. Furthermore, one obtained by adding 5 µL of the aqueous solution of 0.05% (V/V) ethanol to the culture medium and culturing for 24 hours was also prepared. Thereafter, 0.1 mL of a DMSO solution of atmospheric dust (NIST 1648a) was added per 100 mL of the culture medium so that the final concentration of the atmospheric dust in the culture medium was 500 µg/mL, and culturing was further performed for 6 hours. Next, the medium containing atmospheric dust was removed and replaced with a new medium, 0.1 mL of a DMSO solution of atmospheric dust (NIST 1648a) was added per 100 mL of the culture medium so that the final concentration of the atmospheric dust in the culture medium was 500 µg/mL, and culturing was further performed for 18 hours. Thereafter, the cells were stained using the above-mentioned invasion assay kit, and the fluorescence intensity at an excitation wavelength of 480 nm/an absorption wavelength of 570 nm was measured with a microplate reader i-Control (manufactured by Tecan Group Ltd.). One to which atmospheric dust was not added was used as a control.

Table 4 shows the results. Table 4 shows the cell invasion in the atmospheric dust-added group as a relative amount when the fluorescence intensity in the control to which atmospheric dust was not added was 1. In the Na tocopherol phosphate-added group, the cell invasion of cancer cells was reduced as compared to the 0.05% ethanol-added group and the tocopherol acetate-added group. From these results, it was confirmed that the Na tocopherol phosphate suppresses the cell invasion of cancer cells accelerating due to atmospheric dust.

**[Table 4]**

| Sample | | Relative cell invasion |
|---|---|---|
| Atmospheric dust not added | 0.05% Ethanol | 1.00 |
| Atmospheric dust added | 0.05% Ethanol | 2.53 |
| | Na tocopherol phosphate | 1.17 |
| | Tocopherol acetate | 2.08 |

### [Prescription examples]

Prescription examples of the composition for protection against atmospheric pollutants are described below. As Na tocopherol phosphate in the following prescription examples, TPNa (registered trademark) (manufactured by Showa Denko K.K.), which is sodium dl-α-tocopheryl phosphate, is the exemplary example.

### (Prescription Example 1)

Table 5 shows prescription examples of a spreading agent (spray).

**[Table 5]**

| Component | Prescription (mass%) |
|---|---|
| Na tocopherol phosphate | 0.2 |
| Carboxy vinyl polymer | 1 |
| Ethyl alcohol | 30 |
| Phenoxyethanol | 0.2 |
| Water | 68.6 |

### (Prescription Example 2)

Table 6 shows prescription examples of a spreading agent (aerosol).

**[Table 6]**

| Component | Prescription (mass%) |
|---|---|
| Na tocopherol phosphate | 0.2 |
| Ethyl alcohol | 35 |
| Water | 10 |
| Nitrogen gas (propellant) | 54.8 |

### (Prescription Example 3)

Table 7 shows prescription examples of a spreading agent (spray for skin).

**[Table 7]**

| Component | Prescription (mass%) |
|---|---|
| Na tocopherol phosphate | 0.2 |
| Carboxy vinyl polymer | 1 |
| Ethyl alcohol | 25 |
| Titanium oxide | 0.5 |
| Sodium hyaluronate | 0.5 |
| Phenoxyethanol | 0.2 |
| Water | 72.6 |

### (Prescription Example 4)

Table 8 shows prescription examples of a nasal drop.

**[Table 8]**

| Component | Prescription (mass%) |
|---|---|
| Na tocopherol phosphate | 0.2 |
| Carboxy vinyl polymer | 0.5 |
| Sodium chloride | 1 |
| Benzalkonium chloride | 0.2 |
| Ethanol | 0.1 |
| Edetic acid | 0.2 |
| Menthol | Minute amounts |
| Sorbitan sesquioleate | 0.2 |
| Water | 97.1 |

### [Industrial Applicability]

According to the present invention, an agent for protection against atmospheric pollutants which can protect cells from harmful actions due to atmospheric pollutants, and a composition for protection against atmospheric pollutants which contains the above-mentioned agent for protection against atmospheric pollutants are provided.

## Claims

1. An agent for protection against atmospheric pollutants comprising a tocopherol phosphate ester or a salt thereof as an active ingredient.

2. The agent for protection against atmospheric pollutants according to Claim 1, wherein the tocopherol phosphate ester is an α-tocopherol phosphate ester.

3. The agent for protection against atmospheric pollutants according to Claim 1 or 2, wherein the salt of the tocopherol phosphate ester is a sodium salt of the tocopherol phosphate ester.

4. The agent for protection against atmospheric pollutants according to any one of Claims 1 to 3, wherein the agent for protection against atmospheric pollutants promotes expression of an NRF2 gene.

5. The agent for protection against atmospheric pollutants according to any one of Claims 1 to 4, wherein the agent for protection against atmospheric pollutants suppresses production of active oxygen.

6. A composition for protection against atmospheric pollutants comprising:
the agent for protection against atmospheric pollutants according to any one of Claims 1 to 5; and
a pharmaceutically acceptable carrier.

7. The composition for protection against atmospheric pollutants according to Claim 6, wherein a total content of the tocopherol phosphate ester or the salt thereof is 0.1% to 2% by mass.

8. A method for using the composition for protection against atmospheric pollutants according to Claim 6 or 7, the method comprising using the composition for protection against atmospheric pollutants by spraying the composition on a body surface.

9. A method for using the composition for protection against atmospheric pollutants according to Claim 6 or 7, the method comprising using the composition for protection against atmospheric pollutants by applying the composition to a mucous membrane.
